# EUROPÄISCHE PATENTSCHRIFT

(11) **EP 3 194 362 B1**
(45) Veröffentlichungstag und Bekanntmachung des Hinweises auf die Patenterteilung: **10.09.2025**
(21) Anmeldenummer: 15763630.9
(22) Anmeldetag: 18.09.2015
(51) Int. Cl.: C07C 263/10

(54) **VERFAHREN ZUR HERSTELLUNG VON 1,5-PENTANDIISOCYANAT IN DER GASPHASE**
METHOD FOR THE PREPARATION OF 1,5-PENTANDIISOCYANATE IN THE GAS PHASE
PROCÉDÉ DE FABRICATION DE 1,5 DIISOCYANATES DE PENTANE EN PHASE GAZEUSE

(30) Priorität: 19.09.2014 EP 14185564
(43) Veröffentlichungstag der Anmeldung: 26.07.2017
(73) Patentinhaber: Covestro Deutschland AG, 51373 Leverkusen (DE)
(72) Erfinder: SANDERS, Josef, 51375 Leverkusen (DE); EHRIG, Martin, 51373 Leverkusen (DE); HALPAAP, Reinhard, 51519 Odenthal (DE); KELLER-KILLEWALD, Manfred, 41539 Dormagen (DE); SCHYMURA, Armin, 41469 Neuss (DE); WASTIAN, Dietmar, 41542 Dormagen (DE)
(74) Vertreter: Levpat
(86) Internationale Anmeldenummer: PCT/EP2015/071438
(87) Internationale Veröffentlichungsnummer: WO 2016/042125

(56) Entgegenhaltungen:
- EP-A1- 1 555 258
- EP-A1- 2 684 867
- WO-A1-2007/028715
- WO-A1-2009/027232

## Beschreibung

Die Erfindung betrifft ein Verfahren zur Herstellung von 1,5-Pentandiisocyanat (PDI) durch Umsetzung von1,5-Pentandiamin (PDA) mit Phosgen in der Gasphase.

Isocyanate werden in großen Mengen hergestellt und dienen hauptsächlich als Ausgangsstoffe zur Herstellung von Polyurethanen. Da die üblichen monomeren Diisocyanate eine relativ niedrige Molmasse und in der Regel entsprechend hohen Dampfdruck aufweisen, werden insbesondere in der Lackanwendung aus Gründen der Arbeitshygiene daraus hergestellte Polyisocyanate verwendet. Hierbei handelt es sich z.B. um Uretdione, Isocyanurate, Iminooxadiazindione, Biurete, Urethane, Allophanate oder Harnstoffe, die aus den monomeren Diisocyanaten durch Di- und Trimerisierung in der Regel in Gegenwart von Katalysatoren hergestellt werden. Hierzu werden allerdings besonders hohe Anforderungen an die Reinheit der Monomeren gestellt, da die in ihnen üblicherweise enthaltenen Nebenkomponenten die Aktivität der Katalysatoren teilweise stark vermindern. Es müssen dann höhere Katalysator-Konzentrationen oder längere Reaktionszeiten angewendet werden, was die Qualität der resultierenden Polyisocyanate z.B. im Hinblick auf Farbe und Lagerstabilität deutlich verschlechtert.

Es ist daher wünschenswert, dass bereits bei der Herstellung der monomeren Diisocyanate möglichst wenig Nebenkomponenten gebildet werden, um anschließend den Aufwand zu ihrer Entfernung bzw. Minimierung, z. B. durch fraktionierte Destillation, in Grenzen zu halten.

Im Fall von 1,5-Pentandiisocyanat werden insbesondere die chlorhaltigen Nebenkomponenten 5-Chlorpentylisocyanat (CPI), N-Carbamoylpiperidin ("C6-Im") sowie die beiden isomeren N-Carbamoyltetrahydropyridine ("C6-Az") gebildet. Während die Bildung von CPI die Ausbeute vermindert und CPI wegen seiner Monofunktionalität als Kettenabbrecher bei der Weiterverarbeitung stört, beeinträchtigen insbesondere die zum sogenannten HC-Wert (hydrolisierbares Chlor) beitragenden Komponenten C6-Im und C6-Az die Katalyse bei der Weiterverarbeitung von PDI zu Polyisocyanaten, so dass der HC-Wert der zur Herstellung der Polyisocyanate eingesetzten Monomeren in jedem Fall < 100, bevorzugt < 50 ppm liegen sollte.

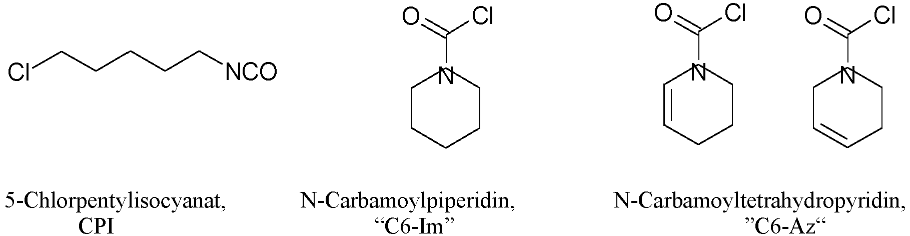

Im PDI, das zur Herstellung von Polyisocyanaten verwendet wird, sollte die Konzentration an CPI < 0,3 % liegen und die Summe der Konzentrationen von C6-Im und C6-Az 400 ppm, bevorzugt 200 ppm, nicht überschreiten. Da die Abtrennung von C6-Im und C6-Az vom PDI, z.B. durch Destillation, sehr schwierig und aufwändig ist, sollte ihre Konzentration auch bereits in den Rohwaren nicht wesentlich höher liegen.

Die Herstellung von 1,5-Pentandiisocyanat (PDI) aus 1,5-Pentandiamin (PDA) ist an sich bekannt und kann phosgenfrei (T. Lesiak, K. Seyda, Journal für Praktische Chemie (Leipzig), 1979, 321 (1), 161 - 163) oder durch Umsetzung mit Phosgen (z.B. W. Siefken, Justus Liebigs Ann. Chem. 562, 1949, S. 25 ff., (S. 122) oder DE 2 625 075 A1 ) erfolgen.

Bei der oben zitierten phosgenfreien Herstellung wird PDA zunächst mit Ameisensäure zum Formamid umgesetzt und dann mit Halologen in Gegenwart von tertiären Aminen zu PDI oxidiert. Nachteilig bei diesem Verfahren ist, dass es sich um ein aufwändiges zweistufiges Verfahren handelt, wobei Nebenprodukte in beträchtlichem Ausmaß gebildet werden. Die hierdurch verursachten Ausbeuteverluste und der erforderliche hohe Reinigungsaufwand vermindern die Wirtschaftlichkeit dieses Verfahrens. DE 2 625 075 A1 beansprucht ein Verfahren zur Herstellung von Carabamidsäurechloriden und Isocyanaten, dadurch gekennzeichnet, dass man Salze von primären Aminen in fester Form in Anwesenheit einer Flüssigkeit bei erhöhter Temperatur in einem Drehrohrofen, einem Schaufeltrockner oder in einem Wirbelbettreaktor mit Phosgen umsetzt. Nachteilig an diesem Verfahren ist, dass es sich auch hier um ein mehrstufiges Verfahren handelt, bei dem in der ersten Stufe zunächst ein Aminsalz in einem Lösungsmittel hergestellt wird, das dann vor der Umsetzung mit Phosgen, z.B. durch Filtration oder Zentrifugation und anschließender Trocknung, wieder entfernt werden muss. Dies ist zeit- und kostenaufwändig und vermindert die Wirtschaftlichkeit dieses Verfahrens.

DE 1 900 514 A1 beschreibt die zweistufige Herstellung von PDI aus Caprolactam durch Überführung in die Hydroxamsäuren und deren anschließende Phosgenierung. Die in dieser Schrift angegebene Ausbeute für die Umwandlung von Caprolactam in PDI beträgt lediglich ca. 32%.

WO 2008/015134 A1 beansprucht ein Verfahren zur Herstellung von PDI, in dem biobasiertes Lysin in PDA überführt wird, welches nachfolgend in PDI überführt wird. Dabei kann die Überführung von PDA in PDI phosgenfrei oder in Gegenwart von Phosgen erfolgen, wobei die letztere Variante in der Flüssigphase oder in der Gasphase erfolgen kann. Eventuell im PDI vorhandene störende Verunreinigungen und Maßnahmen zu ihrer Vermeidung bzw. Minimierung werden nicht erwähnt.

EP 2 684 867 A1 beansprucht 1,5-Pentandiisocyanat (PDI) mit einem Gehalt von 5-400 ppm an den Verbindungen (1) und (2) durch Kalt-Heiss-Phosgenierung von biobasiertem 1,5-Pentandiamin (PDA) oder seines Salzes, ein Verfahren zu seiner Herstellung und damit hergestellte Polyisocyanate.

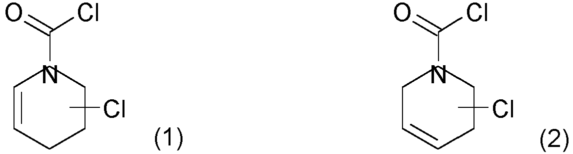

Beschrieben wird darin die Phosgenierung von 1,5-Pentandiamin-Salzen wie z.B. Hydrochloriden in inerten Lösungsmitteln wie z.B. o-Dichlorbenzol , wobei das so erhaltene rohe PDI zur Senkung des Gehalts an Verbindungen (1) und (2) vor der Destillation durch Erhitzen in Gegenwart eines Inertgases wie z.B. Stickstoff und ggf. einer phosphorhaltigen Verbindung wie z.B. Tris(tridecyl)phosphit auf 180-245°C konditioniert wird. Über das Vorhandensein der Nebenkomponente CPI oder ihre Entfernung wird nichts mitgeteilt. Auch dieses Verfahren beinhaltet mehrere Schritte und erfordert lange Reaktionszeiten, was sich ungünstig auf seine Wirtschaftlichkeit auswirkt.

WO 2009/027232 A1 beschreibt allgemein ein Verfahren zur Herstellung von Isocyanaten durch Umsetzung der korrespondierenden Amine mit Phosgen in Gegenwart von mindestens einem Inertmedium in der Gasphase, wobei zwischen die fluiden Ströme von Amin und Phosgen ein Inertmedium dosiert wird. Hierdurch soll die Feststoffablagerung an der Zusammenführung des Amin- und des Phosgenstroms unterdrückt werden. Das Verfahren ist konkret am Beispiel von 1,6-Hexamethylendiisocyanat beschrieben. WO 2009/027232 A1 liefert keinen Hinweis, dass das dortige Verfahren immer noch nachteilig ist aufgrund der entstehenden Anteile an C6-Im und C6-Az und erst recht keinen Hinweis, wie das beschriebene Verfahren zur Vermeidung hoher Anteile dieser Verbindungen zu führen wäre.

Es besteht daher weiterhin ein großer Bedarf an einem einfachen und kostengünstigen Verfahren zur Herstellung von PDI mit ausreichend niedrigen Gehalten an CPI, C6-Im und den beiden isomeren C6-Az, das die Nachteile der Verfahren des Standes der Technik vermeidet.

Überraschend wurde nun gefunden, dass PDI-Rohwaren mit bereits sehr niedrigen Gehalten an CPI, C6-Im und den beiden isomeren C6-Az erhalten werden können, in dem man PDA oberhalb seiner Siedetemperatur unter speziellen Bedingungen wie nachfolgend beschrieben in der Gasphase mit Phosgen umsetzt.

Gegenstand der vorliegenden Erfindung ist ein Verfahren zur Herstellung von 1,5-Pentandiisocyanat (PDI) durch Umsetzung von 1,5-Pentandiamin (PDA) mit Phosgen in der Gasphase, dadurch gekennzeichnet, dass
a) die Gas-Temperaturen sowohl von Phosgen als auch von 1,5-Pentandiamin (PDA) vor Eintritt in den Reaktor im Bereich von 230-320°C liegen und
b) die beiden Eduktströme, sowie ein Inertgasstrom dem Reaktor mittels einer Ringspaltdüse zugeführt werden, wobei der Inertgasstrom über den Ringspalt und damit zwischen den beiden Eduktströmen zugeführt wird,
c) die Eduktströme und der Inertgasstrom sich nach dem Eintritt in den Reaktor mischen und
d) anschließend das Amin und das Phosgen reagieren.

Die Phosgenierung von Aminen in der Gasphase an sich ist bekannt und kann beispielsweise erfolgen, wie beschrieben in EP 0 289 840 B1, EP 1 319 655 A2, EP 1 555 258 A1, EP 1 275 639 A1, EP 1 275 640 A1, EP 1 449 826 A1, EP 1 754 698 B1, DE 10 359 627 A1 oder in der deutschen Patentanmeldung DE 10 2005 042392 A1.

Zum Einsatz kommt technisches PDA mit einer Reinheit > 99% und einem Wassergehalt von < 500 ppm. Es kann durch bekannte Verfahren sowohl aus petrochemisch basierter Herstellung wie auch aus biobasierter Herstellung, z. B. durch Decarboxylierung von Lysin, stammen.PDA aus biobasierter Herstellung ist bevorzugt.

Vor der Durchführung des erfindungsgemäßen Verfahrens wird das PDA verdampft, auf 230°C bis 320°C, vorzugsweise 270°C bis 310°C, erhitzt und dem Reaktor, bevorzugt Rohreaktor, zugeführt. Dabei können dem PDA ein Inertgas wie N₂, He, Ar oder Dämpfe eines inerten Lösungsmittels, z. B. aromatischen Kohlenwasserstoffen mit oder ohne Halogensubstitution, beigemischt werden.

Das bei der Phosgenierung verwendete Phosgen wird vor der Einspeisung in den Reaktor, ebenfalls auf 230°C bis 320°C, vorzugsweise 270°C bis 310°C, erhitzt.

Die beiden Eduktströme und der Inertgasstrom werden dem Reaktor mittels einer Ringspaltdüse, wie sie beispielsweise in EP 1 555 258 A1 beschrieben ist, zugeführt. Diese Patentanmeldung beansprucht ein Verfahren zur Herstellung von Di- und Triisocyanaten in der Gasphase in einem Rohrreaktor, der ein zentrisch in Richtung seiner Rotationsachsachse angeordnetes doppelwandiges Leitrohr aufweist, wobei zwischen der inneren und äußeren Wand dieses doppelwandiges Leitrohres ein konzentrischer Ringspalt ausgebildet ist, die dampfförmigen Di- und/oder Triamine und Phosgen getrennt voneinander auf Temperaturen von 200-600°C erhitzt werden und der Aminstrom über den konzentrischen Ringspalt dem Rohrreaktor zugeführt wird, während Phosgen dem Rohreaktor auf der verbleibenden Querschnittsfläche des Rohrreaktors zugeführt wird. Die Herstellung von 1,5-Pentandiisocyanat (PDI) wird nicht beschrieben, auch finden sich dort keine Hinweise auf Bildung von chlorhaltigen Nebenprodukten oder Maßnahmen zu ihrer Vermeidung bzw. Minimierung.

Bei der Durchführung des erfindungsgemäßen Verfahrens wird ein Rohrreaktor verwendet, der ebenfalls ein zentrisch in Richtung seiner Rotationsachsachse angeordnetes doppelwandiges Leitrohr aufweist, wobei zwischen der inneren und äußeren Wand ein konzentrischer Ringspalt ausgebildet ist. Im Gegensatz zur in der EP 1 555 258 A1 beschriebenen Verfahrensweise wird hier der ggf. durch ein Inertmedium verdünnte, vorerhitzte PDA-Strom mit einer mittleren Geschwindigkeit von 20-150 m/s, vorzugsweise 20-100 m/s, durch das innere Doppelmantelrohr dem Rohrreaktor zugeführt, während das vorerhitzte Phosgen dem Reaktor auf der verbleibenden Querschnittsfläche zwischen dem äußeren Doppelmantelrohr und der inneren Wand des Rohrreaktors mit einer mittleren Strömungsgeschwindigkeit von mindestens 1 m/s vorzugsweise 5-15 m/s zugeführt wird. Zusätzlich werden die beiden Eduktströme beim Eintritt in den Reaktor durch einen zylindermantelförmigen Inertgasstrom getrennt, der nach Vorerhitzung auf ebenfalls 230°C bis 320°C, vorzugsweise 270°C bis 310°C, über den konzentrischen Ringspalt des Doppelmantelrohres dem Rohrreaktor mit einer mittleren Geschwindigkeit von von 20-150 m/s, vorzugsweise 20-100 m/s, zugeführt wird.

Die erfindungsgemäß zu verwendende Ringspaltdüse wird, da der Inertgasstrom, die beiden Eduktströme trennt, im Folgenden auch als Trennspaltdüse oder Stickstofftrennspaltdüse (unter Verwendung von Stickstoff als Inertgas) bezeichnet.

Der Inertgasstrom kann z. B. bestehen aus Stickstoff, Edelgasen wie Helium oder Argon oder aus Dämpfen inerter Lösungsmittel. Bevorzugt ist Stickstoff. Geeignete Lösungsmittel sind beispielsweise aromatische Kohlenwasserstoffe mit oder ohne Halogensubstitution, wie z.B. Chlorbenzol, o-Dichlorbenzol, Toluol, Chlortoluol, Xylol, Chlornaphtalin oder Decahydrodronaphtalin.

Die Mengenströme von gasförmigem PDA und Phosgen werden so gewählt, dass der molare Phosgenüberschuss bezogen auf eine Aminogruppe 30 bis 300 %, bevorzugt 60 bis 200 % beträgt.

Bevorzugt werden im erfindungsgemäßen Verfahren Rohrreaktoren ohne Einbauten und ohne sich bewegende Teile im Innern des Reaktors verwendet. Die Rohrreaktoren bestehen im Allgemeinen aus Stahl, Glas, legiertem oder emailliertem Stahl und sind so dimensioniert, dass unter den Verfahrensbedingungen eine vollständige Umsetzung des PDA mit dem Phosgen ermöglicht wird. Die Gasströme werden wie oben beschrieben über eine Trennspaltdüse an einem Ende des Rohrreaktors in diesen eingeleitet. Die Mischzone wird bevorzugt bei einer Temperatur innerhalb des Bereiches von 230°C bis 320°C, vorzugsweise 270°C bis 310°C, gehalten, wobei diese Temperatur gegebenenfalls durch Beheizung des Rohreaktors aufrecht erhalten werden kann.

Bei der Durchführung des erfindungsgemäßen Verfahrens liegt im allgemeinen der Druck in den Zuleitungen zum Reaktionsraum bei 200 - 3.000 mbar abs., bevorzugt bei 800 - 1.500 mbar abs., und am Ausgang aus dem Reaktionsraum bei 150 - 2. 000 mbar abs., bevorzugt bei 750 - 1440 mbar abs., wobei durch Aufrechterhaltung eines geeigneten Differenzdrucks eine Strömungsgeschwindigkeit innerhalb des Reaktionsraums von 3 bis 120 m/s, bevorzugt 5 bis 75 m/s eingehalten wird. Unter diesen Voraussetzungen herrschen innerhalb des Reaktionsraums im allgemeinen turbulente Strömungsverhältnisse vor.

Die Verweilzeit des Reaktionsgemischs im Reaktor beträgt 0,1 bis 1 s, bevorzugt 0,2 bis 0,5 s. Die Verweilzeit berechnet sich aus dem zeitlichen Durchsatz der Eduktströme, der Reaktordimensionierung und den Reaktionsparametern Druck und Temperatur.

Nach der erfolgten Phosgenierungsreaktion im Reaktionsraum wird das den Reaktionsraum kontinuierlich verlassende, gasförmige Gemisch von dem gebildeten PDI befreit. Dies kann beispielsweise mit Hilfe eines inerten Lösungsmittels erfolgen, dessen Temperatur so gewählt wird, dass sie einerseits oberhalb der Zersetzungstemperatur des dem PDI entsprechenden Carbamidsäurechlorids und andererseits unterhalb der Kondensationstemperatur des PDI und vorzugsweise auch des gegebenenfalls in der Dampfform als Verdünnungsmittel mitverwendeten Lösungsmittels liegt, so dass PDI und Hilfslösungsmittel kondensieren bzw. sich in dem Lösungsmittel lösen, während überschüssiges Phosgen, Chlorwasserstoff und gegebenenfalls als Verdünnungsmittel mitverwendetes Inertgas die Kondensationsstufe bzw. das Lösungsmittel gasförmig durchlaufen. Zur selektiven Gewinnung des PDI aus dem den Reaktionsraum gasförmig verlassenden Gemisch besonders gut geeignet sind bei einer Temperatur von 60 bis 200°C, vorzugsweise 90 bis 170°C, gehaltene Lösungsmittel der oben beispielhaft genannten Art, insbesondere technisches Mono- (MCB) und Dichlorbenzol (ODB). Bervorzugt ist MCB. Denkbare Methoden der selektiven Kondensation des gebildeten Isocyanats aus dem den Reaktor verlassenden Gasgemisch unter Verwendung derartiger Lösungsmittel sind beispielsweise das Durchleiten des Gasgemisches durch das genannte Lösungsmittel oder das Eindüsen des Lösungsmittels (Lösungsmittelnebel) in den Gasstrom (Quenche).

Das die Kondensationsstufe zur Gewinnung des PDI durchlaufende Gasgemisch wird anschließend in an sich bekannter Weise von überschüssigem Phosgen befreit. Dies kann mittels einer Kühlfalle, Absorption in einem bei einer Temperatur von -10°C bis 8°C gehaltenen inerten Lösungsmittel (z.B. Chlorbenzol, MCB, oder Dichlorbenzol, ODB) oder durch Adsorption und Hydrolyse an Aktivkohle erfolgen. Das die Phosgen-Rückgewinnungsstufe durchlaufende Chlorwasserstoffgas kann in an sich bekannter Weise zur Rückgewinnung des zur Phosgensynthese erforderlichen Chlors recykliert werden.

Die Reindarstellung des PDI erfolgt vorzugsweise durch destillative Aufarbeitung der PDI-Rohlösung in dem zur Isocyanatkondensation eingesetzten Lösungsmittel.

Die Vorteile des erfindungsgemäßen Verfahrens sind:
a) Geringe Nebenproduktbildung und dadurch niedrige Gehalte an chlorhaltigen Nebenprodukten bereits in den Rohwaren. Unter Herausrechnung des Lösungsmittels liegen die Konzentrationen von CPI < 0,5 Gew.-%, bevorzugt < 0,3 Gew.-% und die Summe an C6-Im und C6-Az < 400 ppm, bevorzugt < 350 ppm. Dadurch kann der Aufwand bei der nachfolgenden Destillation gering gehalten werden.
b) Vermeidung von festen Ablagerungen an der Reaktorwand und in der Quenche.

Gegenstand der vorliegenden Erfindung ist ein Verfahren zur Herstellung von 1,5-Pentandiisocyanat (PDI) durch Umsetzung von 1,5-Pentandiamin (PDA) mit Phosgen in der Gasphase, dadurch gekennzeichnet, dass
a) die Gas-Temperaturen sowohl von Phosgen als auch von 1,5-Pentandiamin (PDA) vor Eintritt in den Reaktor im Bereich von 230-320°C liegen und
b) die beiden Eduktströme, sowie ein Inertgasstrom dem Reaktor mittels einer Ringspaltdüse zugeführt werden, wobei der Inertgasstrom über den Ringspalt und damit zwischen den beiden Eduktströmen zugeführt wird,
c) die Eduktströme und der Inertgasstrom sich nach dem Eintritt in den Reaktor mischen und
d) anschließend das Amin und das Phosgen reagieren.

In einer zweiten Ausführungsform des Verfahrens liegt die Temperatur des PDA in a) im Bereich von 270°C bis 310°C

In einer dritten Ausführungsform wird das Verfahren gemäß Ausführungsform 1 oder 2 so geführt, dass die Temperatur des Phosgens in a) im Bereich von 270°C bis 310°C liegt.

In einer dritten Ausführungsform wird das Verfahren gemäß einer der Ausführungsformen 1 bis 3 so geführt, dass die Mengenströme von gasförmigem PDA und Phosgen so gewählt werden, dass der molare Phosgenüberschuss bezogen auf eine Aminogruppe 30 bis 300 % beträgt.

In einer vierten Ausführungsform wird das Verfahren gemäß einer der Ausführungsformen 1 bis 3 so geführt, dass die Mengenströme von gasförmigem PDA und Phosgen so gewählt werden, dass der molare Phosgenüberschuss bezogen auf eine Aminogruppe 60 bis 200 % beträgt.

In einer fünften Ausführungsform wird das Verfahren gemäß einer der Ausführungsformen 1 bis 4 so geführt, dass der Druck in den Zuleitungen zum Reaktionsraum bei 200 - 3.000 mbar abs. und der Druck am Ausgang aus dem Reaktionsraum bei 150 - 2.000 mbar abs. liegt.

In einer sechsten Ausführungsform wird das Verfahren gemäß einer der Ausführungsformen 1 bis 4 so geführt, dass der Druck in den Zuleitungen zum Reaktionsraum bei 800 bis 1.500 mbar abs. und der Druck am Ausgang aus dem Reaktionsraum bei 750 bis 1.440 mbar abs. liegt.

In einer siebten Ausführungsform wird das Verfahren gemäß einer der Ausführungsformen 1 bis 6 so geführt, dass die Verweilzeit des Reaktionsgemischs im Reaktor 0,1 bis 1 s beträgt.

In einer achten Ausführungsform wird das Verfahren gemäß einer der Ausführungsformen 1 bis 6 so geführt, dass die Verweilzeit des Reaktionsgemischs im Reaktor 0,2 bis 0,5 s beträgt.

### Beispiele:

### GC-Methode der PDI-Analytik:

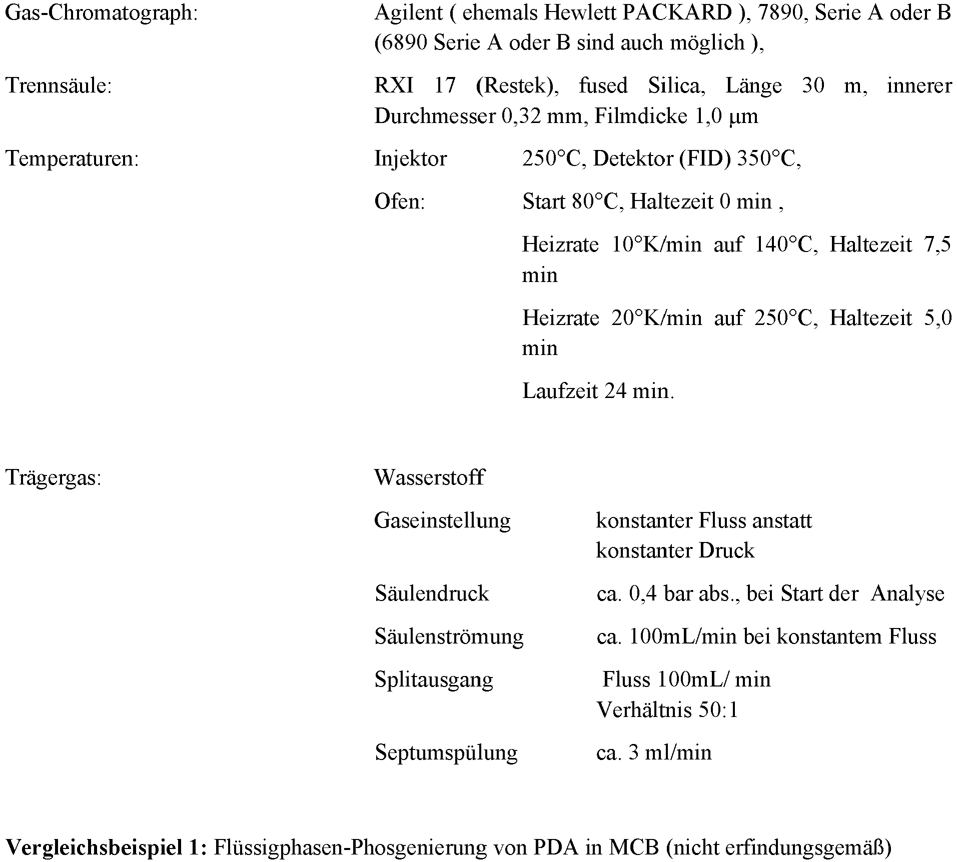

In einem 2 l-Vierhalskolben mit Rührer, Thermometer, Rückflusskühler, Tropftrichter und Gaseinleitungsrohr wurden 463 g MCB vorlegt und hierzu bei -5°C 437 g Phosgen einkondensiert. Unter Rühren und Kühlen wurde dazu innerhalb von 30 Minuten eine Lösung von 75 g PDA in 416 g MCB zugetropft, wobei die Temperatur zwischen 0 - 8°C gehalten wurde. Nach beendeter Zugabe wurde die Kühlung entfernt und die Reaktionsmischung unter weiterem Einleiten von Phosgen allmählich innerhalb von 2 h bis zum Rückfluss erhitzt, wobei im Temperaturbereich von 40-80°C eine stärkere Gasentwicklung auftrat. Anschließend wurde noch weitere 12 h unter Rückfluß phosgeniert. Durch Ausblasen mit Stickstoff wurde die Reaktionsmischung vom Phosgen befreit und filtriert, wobei der Filterrückstand mehrfach mit MCB gewaschen wurde. Der Filterrückstand wurde getrocknet und gewogen während die vereinigten Filtrate durch Vakuum-Destillation mittels Rotavapor weitgehend vom Lösungsmittel befreit wurden. Es wurden erhalten:
Feststoff: 17,3 g; Rohlösung: 97,7 g enthaltend 9% MCB
Ausbeute: 56,7% der Th. PDI

GC-Analytik (MCB herausgerechnet, Flächenprozent ( Fl.-%)):

| | |
|---|---|
| CPI | 1,432 |
| C6-Az | 0,409 |
| C6-Im | 0,000 |
| PDI | 98,159 |

### Vergleichsbeispiel 2: Flüssigphasen-Phosgenierung von PDA in ODB (nicht erfindungsgemäß)

Analog Vergleichsbeispiel 1 wurden 75 g PDA in ODB umgesetzt, wobei Lösungsmittelmenge, Reaktionszeiten und -temperaturen gleich gehalten wurden. Es wurden erhalten:
Feststoff: 14,8 g; Rohlösung: 85,5 g enthaltend 17% ODB
Ausbeute: 44,0% der Th. PDI

GC-Analytik (MCB herausgerechnet, Fl.-%):

| | |
|---|---|
| CPI | 4,047 |
| C6-Az | 0,000 |
| C6-Im | 0,508 |
| PDI | 95,445 |

### Vergleichsbeispiel 3: Gasphasen-Phosgenierung von PDA bei 340°C mit Koaxialdüse (einfache Glattstrahldüse) -(nicht erfindungsgemäß)

In einer Anlage zur Gasphasen-Phosgenierung mit einer Aminverdampfungsstufe, einem Rohreaktor (L: 1770 mm, Innendurchmesser 37 mm) mit einer auf der Reaktorachse angeordneten Koaxialdüse (Innendurchmesser 6,5 mm) und einer nachgeschalteten Isocyanatkondensationsstufe wurden bei einem Druck von 1300 mbar abs. gemessen am Ende der Isocyanatkondensationsstufe, kontinuierlich 6,88 kg/h PDA unter Einleitung eines Stickstoffstroms von 0,138 kg/h verdampft, auf 340°C überhitzt und über die Koaxialdüse dem Reaktor zugeführt. Gleichzeitig wurden parallel dazu 36,6 kg/h Phosgen auf 340°C erhitzt und auf dem von der Düse freigelassenen Ringraum ebenfalls dem Reaktor kontinuierlich zugeführt, in welchem die beiden Eduktströme gemischt und zur Reaktion gebracht wurden. Dabei beträgt die Geschwindigkeit des Gasstroms im Reaktor ca. 6,8 m/s und das Geschwindigkeitsverhältnis von Amin-/Stickstoff- zu Phosgenstrom 5,9. Nach einer mittleren Verweilzeit im Reaktor von 0,26 s wurde der das Reaktionsprodukt PDI enthaltende Gasstrom durch Einspritzkühlung mit Monochlorbenzol abgekühlt und kondensiert, wobei die Temperatur der flüssigen Phase in der Quenche ca. 90°C betrug. Bereits nach 4 h musste die Anlage wegen Druckanstiegs aufgrund von Fouling an der Düse und im Reaktor abgefahren werden.

Die GC-Analytik der erhaltenen Rohlösung zeigte folgende Zusammensetzung (MCB herausgerechnet, Fl.-%):

| | |
|---|---|
| CPI | 0,499 |
| C6-Az | 0,195 |
| C6-Im | 3,893 |
| PDI | 95,414 |

### Vergleichsbeispiel 4: Gasphasen-Phosgenierung von PDA bei 310°C mit Koaxialdüse (nicht erfindungsgemäß)

Die Phosgenierung wurde wie in Beispiel 3 beschrieben durchgeführt, wobei sowohl das mit Stickstoff verdünnte gasförmige PDA wie auch das Phosgen vor Eintritt in den Reaktor auf 310°C erhitzt wurden. Dabei betrug die Geschwindigkeit des Gasstroms im Reaktor ca. 6,5 m/s, das Geschwindigkeitsverhältnis von Amin-/Stickstoff- zu Phosgenstrom 6,0 und die mittlere Verweilzeit im Reaktor von 0,27 s. Hier musste die Anlage nach 7 h wegen Druckanstiegs aufgrund von Fouling an der Düse und im Reaktor abgefahren werden.

Die GC-Analytik der erhaltenen Rohlösung zeigte folgende Zusammensetzung (MCB herausgerechnet, Fl.-%):

| | |
|---|---|
| CPI | 0,823 |
| C6-Az | 0,098 |
| C6-Im | 1,060 |
| PDI | 98,019 |

### Vergleichsbeispiel 5: Gasphasen-Phosgenierung von PDA bei 340°C mit Stickstoff-Trennspaltdüse (nicht erfindungsgemäß)

In einer Anlage zur Gasphasen-Phosgenierung mit einer Aminverdampfungsstufe, einem Rohreaktor (L: 1770 mm, Innendurchmesser 37 mm) mit einer auf der Reaktorachse angeordneten Trennspaltdüse (Innendurchmesser 6,5 mm, Trennpalt: Innendurchmesser 6,5 mm, Außendurchmesser 8,5 mm) und einer nachgeschalteten Isocyanatkondensationsstufe wurden bei einem Druck von 1300 mbar abs. gemessen am Ende der Isocyanatkondensationsstufe, kontinuierlich 8,46 kg/h PDA verdampft, auf 340°C überhitzt und über die innere Zentraldüse dem Reaktor zugeführt. Gleichzeitig wurden parallel dazu 1,48 kg Stickstoff und 45 kg Phosgen auf 310°C erhitzt und über den Trennspalt (Stickstoff) bzw. über den von der Düse freigelassenen Ringraum (Phosgen) ebenfalls dem Reaktor kontinuierlich zugeführt, in welchem die beiden Eduktströme gemischt und zur Reaktion gebracht wurden. Dabei betrug die Geschwindigkeit des Gasstroms im Reaktor ca. 8,9 m/s und das Geschwindigkeitsverhältnis von Amin-/Stickstoff- zu Phosgenstrom 5,59. Nach einer mittleren Verweilzeit im Reaktor von 0,20 s wurde der das Reaktionsprodukt PDI enthaltende Gasstrom durch Einspritzkühlung mit Monochlorbenzol abgekühlt und kondensiert, wobei die Temperatur der flüssigen Phase in der Quenche ca. 90°C betrugt. Die Anlage konnte über einen Zeitraum von 60 h problemlos betrieben werden. Danach stieg der Druck allmählich an, so dass die Anlage nach 66 h aufgrund von Fouling im Reaktor abgefahren werden musste.

Die GC-Analytik der erhaltenen Rohlösung zeigte folgende Zusammensetzung (MCB herausgerechnet, Fl.-%):

| | |
|---|---|
| CPI | 0,423 |
| C6-Az | 0,098 |
| C6-Im | 0,360 |
| PDI | 99,119 |

### Beispiel 1: Gasphasen-Phosgenierung von PDA bei 310°C mit Stickstoff-Trennspaltdüse (erfindungsgemäß)

Die Phosgenierung wurde wie in Vergleichsbeispiel 5 beschrieben durchgeführt, wobei PDA, Stickstoff und Phosgen vor Eintritt in den Reaktor auf 310°C erhitzt wurden. Dabei betrug die Geschwindigkeit des Gasstroms im Reaktor ca. 8,5 m/s, das Geschwindigkeitsverhältnis von Amin-/Stickstoff- zu Phosgenstrom 6,0 und die mittlere Verweilzeit im Reaktor 0,21 s. Die Anlage konnte über einen Zeitraum von 100 h problemlos betrieben werden. Nach Abfahren und Öffnen der Anlage wiesen Düse und Reaktor keine Verschmutzungen auf.

Die GC-Analytik der erhaltenen Rohlösung zeigte folgende Zusammensetzung (MCB herausgerechnet, Fl.-%):

| | |
|---|---|
| CPI | 0,286 |
| C6-Az | 0,032 |
| C6-Im | 0,004 |
| PDI | 99,678 |

Bei der Flüssigphasen-Phosgenierung von PDA (Basenphosgenierung) in MCB und ODB (Vergleichsbeispiel 1 u. 2) werden sehr schlechte Ausbeuten erhalten und es entstehen sehr hohe Anteile an CPI.

Bei der Gasphasen-Phosgenierung mit der Koaxialdüse (einfache Glattstrahldüse) entstehen bei 340°C (Vergleichsbeispiel 3) prohibitiv hohe Anteile an C6-Im. Auch bei 310°C (Vergleichssbeispiel 4) liegen die C6-Im Anteile immer noch relativ hoch. Außerdem werden bei den Vergleichsbeispielen 3 und 4 aufgrund von Fouling an Düse und Reaktor nur sehr kurze Anlagen-Laufzeiten erreicht.

Bei der Gasphasen-Phosgenierung mit der Stickstoff-Trennspaltdüse entstehen bei 340°C (Vergleichsbeispiel 5) immer noch hohe C6-Im Anteile. Die Laufzeit ist zwar verbessert, aber immer noch unbefriedigend.

Beispiel 1 zeigt, dass durch die Kombination der Verwendung einer Stickstoff-Trennspaltdüse und Edukt-Temperaturen von 310°C PDA, Stickstoff und Phosgen vor Eintritt in den Reaktor die Anteile an chlorhaltigen Nebenprodukten deutlich vermindert werden können und auch nach längerer Anlagenlaufzeit kein Fouling mehr auftritt.

## Patentansprüche

1. Verfahren zur Herstellung von 1,5-Pentandiisocyanat (PDI) durch Umsetzung von 1,5-Pentandiamin (PDA) mit Phosgen in der Gasphase, **dadurch gekennzeichnet, dass**
a) die Gas-Temperaturen sowohl von Phosgen als auch von 1,5-Pentandiamin (PDA) vor Eintritt in den Reaktor im Bereich von 230-320°C liegen und
b) die beiden Eduktströme, sowie ein Inertgasstrom dem Reaktor mittels einer Ringspaltdüse zugeführt werden, wobei der Inertgasstrom über den Ringspalt und damit zwischen den beiden Eduktströmen zugeführt wird,
c) die Eduktströme und der Inertgasstrom sich nach dem Eintritt in den Reaktor mischen und
d) anschließend das Amin und das Phosgen reagieren.

2. Verfahren zur Herstellung von 1,5-Pentandiisocyanat (PDI) gemäß Anspruch 1, wobei technisches PDA mit einer Reinheit > 99% und einem Wassergehalt von < 500 ppm verwendet wird.

3. Verfahren zur Herstellung von 1,5-Pentandiisocyanat (PDI) gemäß Anspruch 2, wobei das PDA aus biobasierter Herstellung stammt.

4. Verfahren zur Herstellung von 1,5-Pentandiisocyanat (PDI) gemäß einem der Ansprüche 1 bis 3, wobei die Temperatur des PDA in a) im Bereich von 270°C bis 310°C liegt.

5. Verfahren zur Herstellung von 1,5-Pentandiisocyanat (PDI) gemäß einem der Ansprüche 1 bis 4, wobei die Temperatur des Phosgens in a) im Bereich von 270°C bis 310°C liegt.

6. Verfahren zur Herstellung von 1,5-Pentandiisocyanat (PDI) gemäß einem der Ansprüche 1 bis 5, wobei die Mengenströme von gasförmigem PDA und Phosgen so gewählt werden, dass der molare Phosgenüberschuss bezogen auf eine Aminogruppe 30 bis 300 % beträgt.

7. Verfahren zur Herstellung von 1,5-Pentandiisocyanat (PDI) gemäß einem der Ansprüche 1 bis 6, wobei die Ringspaltdüse durch einen Rohrreaktor gebildet wird, der ein zentrisch in Richtung seiner Rotationsachsachse angeordnetes doppelwandiges Leitrohr aufweist, wobei zwischen der inneren und äußeren Wand ein konzentrischer Ringspalt ausgebildet ist.

8. Verfahren zur Herstellung von 1,5-Pentandiisocyanat (PDI) gemäß einem der Ansprüche 1 bis 7, wobei der Inertgasstrom in c) aus Stickstoff, einem Edelgas, Dämpfen eines inerten Lösungsmittels oder Mischungen dieser Inertgase besteht.

9. Verfahren zur Herstellung von 1,5-Pentandiisocyanat (PDI) gemäß einem der Ansprüche 1 bis 8, wobei der Druck in den Zuleitungen zum Reaktionsraum bei 200 - 3.000 mbar abs. liegt.

10. Verfahren zur Herstellung von 1,5-Pentandiisocyanat (PDI) gemäß einem der Ansprüche 1 bis 9, wobei der Druck am Ausgang aus dem Reaktionsraum bei 150 - 2. 000 mbar abs. liegt.

11. Verfahren zur Herstellung von 1,5-Pentandiisocyanat (PDI) gemäß einem der Ansprüche 1 bis 10, wobei die Verweilzeit des Reaktionsgemischs im Reaktor 0,1 bis 1 s beträgt.

## Claims

1. Process for preparing pentane 1,5-diisocyanate (PDI) by reacting pentane-1,5-diamine (PDA) with phosgene in the gas phase, **characterized in that**
a) the gas temperatures both of phosgene and of pentane-1,5-diamine (PDA) prior to entry into the reactor are in the range of 230-320°C and
b) the two reactant streams, and also an inert gas stream, are supplied to the reactor by means of an annular gap nozzle, the inert gas stream being supplied through the annular gap and hence between the two reactant streams,
c) the reactant streams and the inert gas stream mix after entry into the reactor and
d) then the amine and the phosgene react.

2. Process for preparing pentane 1,5-diisocyanate (PDI) according to Claim 1, wherein technical grade PDA having a purity of > 99% and a water content of < 500 ppm is used.

3. Process for preparing pentane 1,5-diisocyanate (PDI) according to Claim 2, wherein the PDA originates from biobased production.

4. Process for preparing pentane 1,5-diisocyanate (PDI) according to any of Claims 1 to 3, wherein the temperature of the PDA in a) is in the range from 270°C to 310°C.

5. Process for preparing pentane 1,5-diisocyanate (PDI) according to any of Claims 1 to 4, wherein the temperature of the phosgene in a) is in the range from 270°C to 310°C.

6. Process for preparing pentane 1,5-diisocyanate (PDI) according to any of Claims 1 to 5, wherein the flow rates of gaseous PDA and phosgene are chosen such that the molar phosgene excess based on one amino group is 30% to 300%.

7. Process for preparing pentane 1,5-diisocyanate (PDI) according to any of Claims 1 to 6, wherein the annular gap nozzle is formed by a tubular reactor having a twin-wall guide tube arranged centrally in the direction of its axis of rotation, wherein a concentric annular gap is formed between the inner and outer walls.

8. Process for preparing pentane 1,5-diisocyanate (PDI) according to any of Claims 1 to 7, wherein the inert gas stream in c) consists of nitrogen, a noble gas, vapours of an inert solvent or mixtures of these inert gases.

9. Process for preparing pentane 1,5-diisocyanate (PDI) according to any of Claims 1 to 8, wherein the pressure in the inlets to the reaction space is 200-3000 mbar abs.

10. Process for preparing pentane 1,5-diisocyanate (PDI) according to any of Claims 1 to 9, wherein the pressure at the outlet from the reaction space is 150-2000 mbar abs.

11. Process for preparing pentane 1,5-diisocyanate (PDI) according to any of Claims 1 to 10, wherein the dwell time of the reaction mixture in the reactor is 0.1 to 1 s.

## Revendications

1. Procédé de préparation de diisocyanate de 1,5-pentane (PDI) par transformation de 1,5-pentanediamine (PDA) avec du phosgène en phase gazeuse, **caractérisé en ce que**
a) les températures des gaz, tant celle du phosgène que celle de la 1,5-pentanediamine (PDA) avant l'entrée dans le réacteur se situent dans la plage de 230-320°C et
b) les deux flux de produit départ ainsi qu'un flux de gaz inerte sont introduits dans le réacteur au moyen d'une buse à fente annulaire, le flux de gaz inerte étant introduit par l'intermédiaire de la fente annulaire et donc entre les deux flux de produit de départ,
c) les flux de produit de départ et le flux de gaz inerte se mélangent après l'entrée dans le réacteur et
d) l'amine et le phosgène réagissent ensuite.

2. Procédé de préparation de diisocyanate de 1,5-pentane (PDI) selon la revendication 1, de la PDA de qualité technique présentant une pureté > 99% et une teneur en eau < 500 ppm étant utilisée.

3. Procédé de préparation de diisocyanate de 1,5-pentane (PDI) selon la revendication 2, la PDA provenant d'une préparation biosourcée.

4. Procédé de préparation de diisocyanate de 1,5-pentane (PDI) selon l'une des revendications 1 à 3, la température de la PDA dans a) étant située dans la plage de 270°C à 310°C.

5. Procédé de préparation de diisocyanate de 1,5-pentane (PDI) selon l'une des revendications 1 à 4, la température du phosgène dans a) étant située dans la plage de 270°C à 310°C.

6. Procédé de préparation de diisocyanate de 1,5-pentane (PDI) selon l'une des revendications 1 à 5, les flux massiques de la PDA et du phosgène gazeux étant choisis de telle sorte que l'excès molaire de phosgène par rapport à un groupe amino est de 30 à 300%.

7. Procédé de préparation de diisocyanate de 1,5-pentane (PDI) selon l'une des revendications 1 à 6, la buse à fente annulaire étant formée par un réacteur tubulaire qui présente un tube de guidage à double paroi agencé de manière centrée dans la direction de son axe de rotation, une fente annulaire concentrique étant réalisée entre la paroi interne et la paroi externe.

8. Procédé de préparation de diisocyanate de 1,5-pentane (PDI) selon l'une des revendications 1 à 7, le flux de gaz inerte dans c) étant constitué d'azote, d'un gaz noble, de vapeurs d'un solvant inerte ou de mélanges de ces gaz inertes.

9. Procédé de préparation de diisocyanate de 1,5-pentane (PDI) selon l'une des revendications 1 à 8, la pression dans les conduites d'alimentation vers la chambre de réaction étant située à 200 - 3000 mbars abs.

10. Procédé de préparation de diisocyanate de 1,5-pentane (PDI) selon l'une des revendications 1 à 9, la pression à la sortie de la chambre de réaction étant située à 150 - 2000 mbars abs.

11. Procédé de préparation de diisocyanate de 1,5-pentane (PDI) selon l'une des revendications 1 à 10, la durée de séjour du mélange réactionnel dans le réacteur représentant 0,1 à 1 s.
